# EUROPEAN PATENT APPLICATION

(11) **EP 4 850 622 A1**
(43) Date of publication of application: **09.12.2026**
(21) Application number: 25181484.4
(22) Date of filing: 06.06.2025
(51) Int. Cl.: A61B 18/00, A61B 18/14, A61B 18/12, A61B 5/251

(54) **A CATHETER-BASED SYSTEM AND METHOD OF USE**

(71) Applicant: AF-ABLATION S.R.L., 20121 Milan (MI) (IT)
(72) Inventor: Pappone, Carlo, Milan (IT)
(74) Representative: Bird & Bird Società tra Avvocati S.r.l.

(57) **Abstract**

The innovative catheter-based system (1000, 2000) configured for simultaneous mapping and ablation of at least a target area of a cardiac tissue wherein the catheter based-system (1000, 2000) comprises: a catheter (100, 200), including a mapping and ablator device (110, 210) disposed in a distal portion of the catheter, a radiofrequency (RF) generator (200); a pulsed field (PFA) generator (300); a control unit (400) configured to switch between the radiofrequency (RF) generator and the pulsed field ablation (PFA) generator, wherein the mapping and ablator device (110, 210) comprises a plurality of flexible and irrigated electrodes (120, 220). Moreover the method (3000) of use of a catheter-based system for mapping and ablating a cardiac or epicardial target area of a patient to be treated, wherein the catheter-based system comprising a catheter including a mapping and ablator device

## Description

### TECHNICAL FIELD

The subject-matter disclosed herein relates to a catheter- based system in particular a catheter-based system enables simultaneous mapping and ablation solution, preferably of a cardiac o epicardial tissue.

### BACKGROUND ART

Catheter ablation has become a cornerstone in the treatment of arrhythmias and cardiac autoimmunity over the years, several ablation modalities have been developed, each with its own strengths and limitations.

Radiofrequency (=RF) ablation remains the most widely used technique. It typically employs cylindrical electrodes to deliver point-by-point energy in a unipolar configuration, forming an electrical circuit with a reference electrode on the patient's skin. While this method is versatile and applicable to a broad range of arrhythmia lesions, it is often criticized for its low efficiency and non-selective tissue damage, which can affect both the target and surrounding structures.

A critical technical component of RF ablation is active irrigation. During energy delivery, the electrode-tissue interface can reach high temperatures, increasing the risk of coagulum formation, tissue charring, and steam pops.

Cryoballoon ablation offers a more efficient approach by using a fusiform balloon filled with cryogen to induce irreversible cryodamage at the pulmonary vein ostia. It allows for trial ablation to verify positioning, but its fixed balloon size limits adaptability to anatomical variations. Moreover, it requires continuous fluoroscopic guidance, and in cases of incomplete ablation, additional point-by-point RF ablation is often needed-raising both procedural complexity and cost.

Pulsed electric field (=PEF) ablation is a newer modality that offers high tissue selectivity and rapid lesion formation. However, it is associated with significant patient discomfort during energy delivery and suffers from poor electrode-tissue contact due to the rigidity of current multipolar catheter designs. This often necessitates repeated repositioning and ablation, reducing procedural efficiency and long-term success.

Several patents have attempted to address these challenges, but each falls short in key areas:
- US11246655B2 introduces flexible electrodes but lacks integrated irrigation and does not support dual energy modalities or anti-distortion features;
- US10492729B2 focuses on high-density mapping but does not integrate ablation capabilities or modular electrode configurations;
- US11383078B2 improves electrode-tissue contact but omits multi-lumen irrigation and flexible, distortion-resistant designs;
- US11596470B2 emphasizes irrigation but does not address the need for flexible, multifunctional electrodes or dual energy compatibility;
- EP4521874 proposes general catheter improvements but lacks modularity, dual energy support, and advanced structural features like helical micro-cuts.

The preceding discussion highlights the ongoing evolution in the field of catheter-based cardiac interventions and underscores the need for further innovation in catheter design, electrode placement and fixation within the catheter, and overall flexibility. Such advancements are essential to overcome the inherent limitations of current technologies and to enable truly adaptive, high-resolution epicardial mapping and therapy.

It would be desirable to develop an innovative catheter-based system specifically designed as a dual-energy mapping and ablation solution, capable of delivering both pulsed field ablation (PFA) and radiofrequency (RF) energy. Such a system should enable the operator to selectively administer PFA and/or RF energy, either independently or simultaneously, and to seamlessly switch between the two energy modalities using the same catheter platform.

This capability would allow for precise targeting of specific tissue regions within the heart, adapting the energy type to the anatomical and electrophysiological characteristics of the substrate. For example, PFA could be used for its high tissue selectivity and non-thermal mechanism, ideal for preserving adjacent structures such as the esophagus or phrenic nerve, while RF could be employed for deep, transmural lesions in areas requiring more extensive ablation.

### SUMMARY

It would be desirable to have an innovative catheter-based system configured for simultaneous mapping and ablation of at least a target area of a cardiac tissue wherein the catheter based-system comprises: a catheter including a mapping and ablator device disposed in a distal portion of the catheter; a radiofrequency (=RF) generator; a pulsed field (=PFA) generator; a control unit configured to switch between the radiofrequency (RF) generator and the pulsed field ablation (PFA) generator, wherein the mapping and ablator device comprises a plurality of flexible and irrigated electrodes.

The invention further relates to an innovative method of use of a innovative catheter-based system with an innovative steps of: advancing the catheter into a cardiac chamber or epicardial space; positioning and conforming the mapping and ablator device to a cardiac or epicardial target area; delivering energy through a plurality of flexible and irrigated electrodes and simultaneously acquiring electrograms; switching between a radiofrequency (RF) generator and a pulsed field (PFA) generator based on data collected and processed by a control unit.

### BRIEF DESCRIPTION OF THE DRAWINGS

A more complete appreciation of the disclosed embodiments of the invention and many of the attendant advantages thereof will be readily obtained as the same becomes better understood by reference to the following detailed description when considered in connection with the accompanying drawings, wherein:
Fig. 1 shows a schematic view of an innovative catheter- based system;
Fig. 2 show a prospective view of a preferred embodiment of an innovative mapping and ablator device of the innovative catheter- based system of Fig. 1;
Figs. 2A show a prospective view of a second embodiment of an innovative mapping and ablator device of Fig. 2;
Figs. 3 and 4 show a prospective view of a detailed features of a flexible electrode of the innovative mapping and ablator device of Fig. 2;
Fig. 5 shows a prospective view of a detailed features of a flexibly railing of the innovative mapping and ablator device of Fig. 2;
Figs. 6 and 7 show a prospective view of a third embodiment of a innovative mapping and ablator device of the innovative catheter- based system of Fig. 1;
Figs. 8, and 9 show a prospective view of a detailed features of an electrode inserted in the flexibly rail of the innovative mapping and ablator device of Fig. 6;
Figs. 10A, 10B and 10C show a view of embodiments of an electrode of the innovative mapping and ablator device of Fig. 6;
Fig. 11 show prospective view of a internal structure of a component of the innovative mapping and ablator device of Fig. 6
Fig.12 shows a cross-section view of a flexible rail with electrode in place in the innovative catheter- based system of Fig. 1;
Fig. 13 shows a flow chart of an embodiment of a step of an innovative method of use of the innovative catheter- based system of Fig. 1.

### DETAILED DESCRIPTION OF EMBODIMENTS

The subject-matter disclosed herein relates to an innovative catheter-based system, i.e. typically dual energy catheter-based system designed to perform simultaneous mapping and ablation of targeted cardiac tissue areas, offering a highly integrated and efficient approach to electrophysiological procedures. It includes a catheter equipped at its distal end with a mapping and ablation device composed of multiple flexible and irrigated electrodes. These electrodes are strategically arranged along a series of symmetrically positioned flexible railings that run parallel to the catheter's longitudinal axis. Each electrode features a stadium-shaped or semi-flat cross-section, optimized for stable tissue contact and uniform energy delivery. To enhance flexibility and ensure effective irrigation, the outer surface of each electrode is engineered with precision micro-cuts that allow fluid to pass through while maintaining structural integrity.

The catheter-based system is powered by both a radiofrequency generator and a pulsed field ablation generator, and it incorporates a control unit capable of switching between the two energy sources. This dual-energy capability enables clinicians to adapt the ablation strategy in real time, using either RF or PFA independently or in combination, depending on the tissue characteristics and procedural goals.

In particular, the system is capable of dynamically switching between the two energy modalities based on real-time tissue responses detected during the procedure, thereby optimizing treatment effectiveness and reducing the risk of collateral damage The ability to deliver both energy types through the same catheter not only reduces the need for device exchanges but also improves procedural efficiency, precision, and safety, also where different tissue responses are required.

Reference now will be made in detail to embodiments of the disclosure, an example of which is illustrated in the drawings. Each example is provided by way of explanation of the disclosure, not limitation of the disclosure. In fact, it will be apparent to those skilled in the art that various modifications and variations can be made in the present disclosure without departing from the scope or spirit of the disclosure. In the following description, similar reference numerals are used for the illustration of figures of the embodiments to indicate elements performing the same or similar functions. Moreover, for clarity of illustration, some references may not be repeated in all the figures.

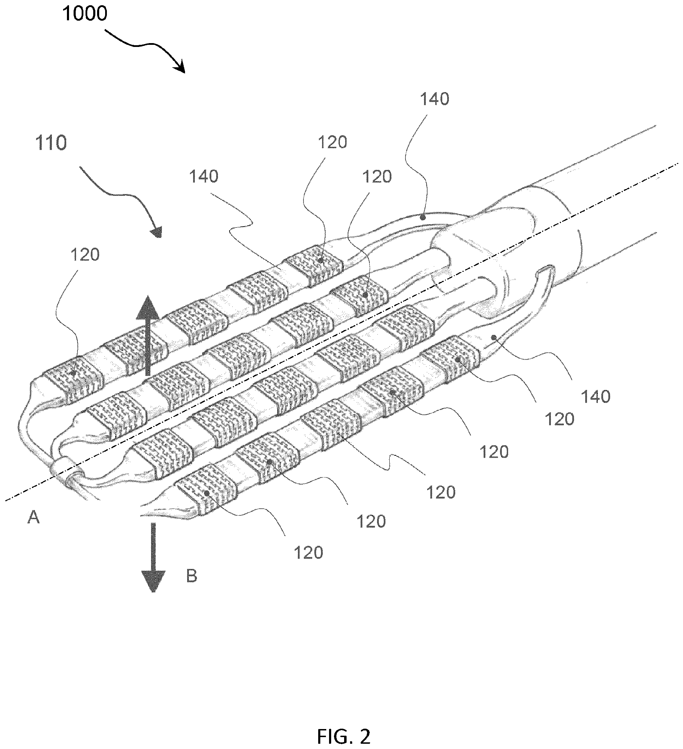

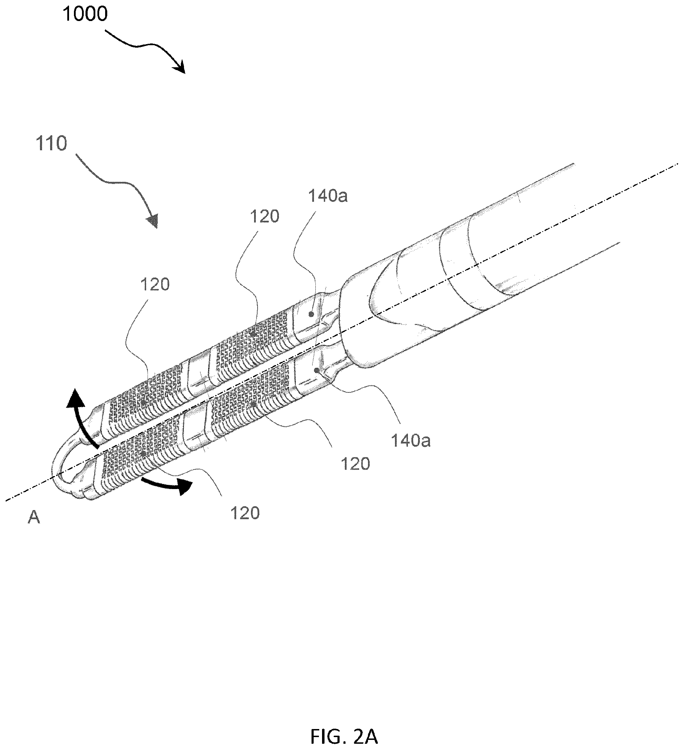

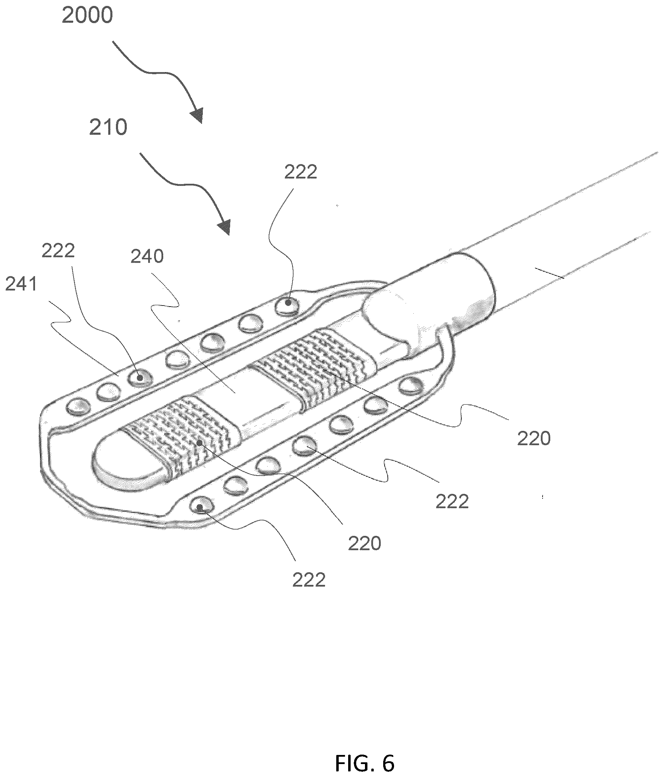

In Figures 2, 2A and 6 are schematically shown yhe embodiments of an innovative catheter -based system. The innovative catheter -based system is generally indicated with reference numeral 1000 in Figs. 2, 2A and 2000 in Fig. 6.

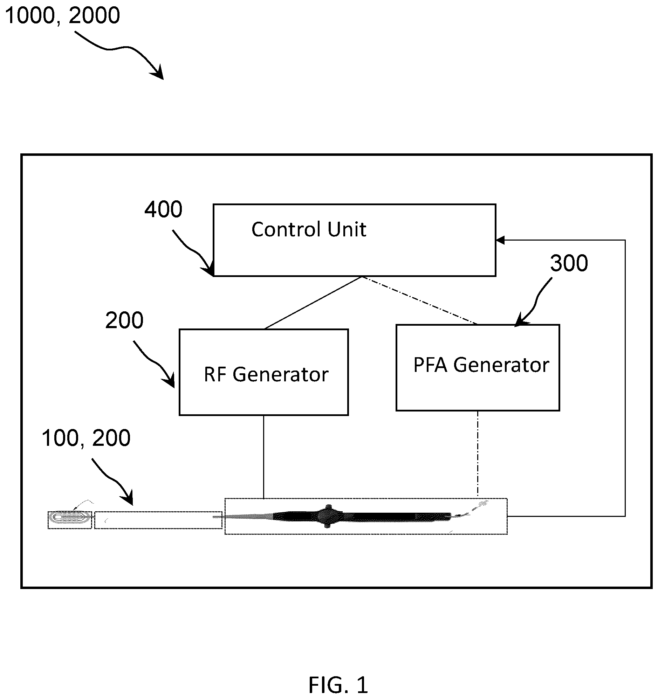

Considering Fig. 1 catheter-based system 1000, 2000 is configured for simultaneous mapping and ablation of at least a target area of a cardiac tissue wherein the catheter based-system 1000, 2000 comprises: a catheter 100, 200, a radiofrequency (RF) generator 200; a pulsed field (PFA) generator 300; a control unit 400 configured to switch between the radiofrequency (RF) generator and the pulsed field ablation (PFA) generator.

Figs. 2, 2A and 6 shows for example and without limitation that the catheter 100, 200 include a mapping and ablator device 110, 210 disposed in a distal portion of the catheter, advantageously the mapping and ablator device 110, 210 comprises a plurality of flexible and irrigated electrodes 120, 220.

Advantageously the control unit 400 is configured to selectively switch between the radiofrequency (RF) generator and the pulsed field ablation (PFA) generator, thereby enabling the delivery of the selected energy modality to the mapping and ablation device located at the distal portion of the catheter 100, based on data collected and processed by the control unit 400.

According to first embodiment shown in Fig. 2 the mapping and ablator device 110 comprises: the plurality of flexible and irrigated electrodes 120; a plurality of flexibly railings 140 arranged symmetrically and parallel to the axis (A) of the catheter 100; the plurality of flexible and irrigated electrodes 120, preferably integrated, along the plurality of flexibly railings 140.

Advantageously the catheter 100, 200 with the mapping and ablator device 110, 210 is configured for simultaneous high-density electrogram acquisition and ablation from the same plurality of electrodes.

Advantageously each of the plurality of flexible and irrigated electrodes 120 has a stadium or semi-flat cross section enhancing conformance to epicardial tissue or cardiac tissue.

Fig. 2A shows a second embodiment of the catheter-based system 1000 comprising a linear catheter wherein the plurality of flexibly railings 140 is two flexibly railings 140a arranged symmetrically and parallel to the axis (A) of the catheter 100 wherein the plurality of flexible and irrigated electrodes 120 disposed along two plurality of flexibly railings 140a.

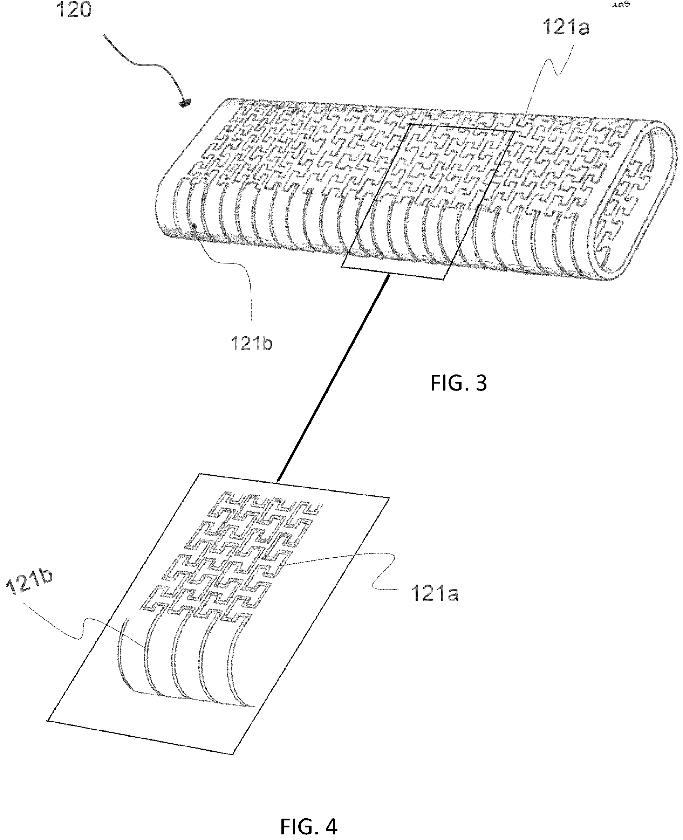

As shown in Figs. 3 and 4 each of the plurality of flexible and irrigated electrodes 120 has at least a cut on the outer surface of each electrode 120 to promote flexibility and allow irrigation of each electrode 120, preferably at least the cut of the plurality of flexible and irrigated electrodes 120 have a diameter d with 0,40≤d≤ 1mm.

It is to be noted that the shape of at least the cut follows a repeating geometric pattern 121a on the bottom portion of the outer surface of each electrode 120, wherein the geometric pattern 121a is preferably helical or H shaped, to prevent stretch, distortion and kerf closure for irrigation and ensure stable irrigation channels during cardiac motion, promoting flexibility and to pass irrigation.

Therefore, the shape of at least the cut follows a linear pattern 121b on the lateral portion of the outer surface of each electrode 120, to increase up/down (B) movement.

As indicated in Fig. 2 the plurality of flexibly railings 140 has a stadium or semi-flat cross section, enhancing conformance to epicardial tissue, in particular to facilitate flexing perpendicularly, bi-directional or radial motion to the catheter shaft, to conform to different epicardium surfaces, to move in-sync with cardiac motion and minimize horizontal or lateral curve.

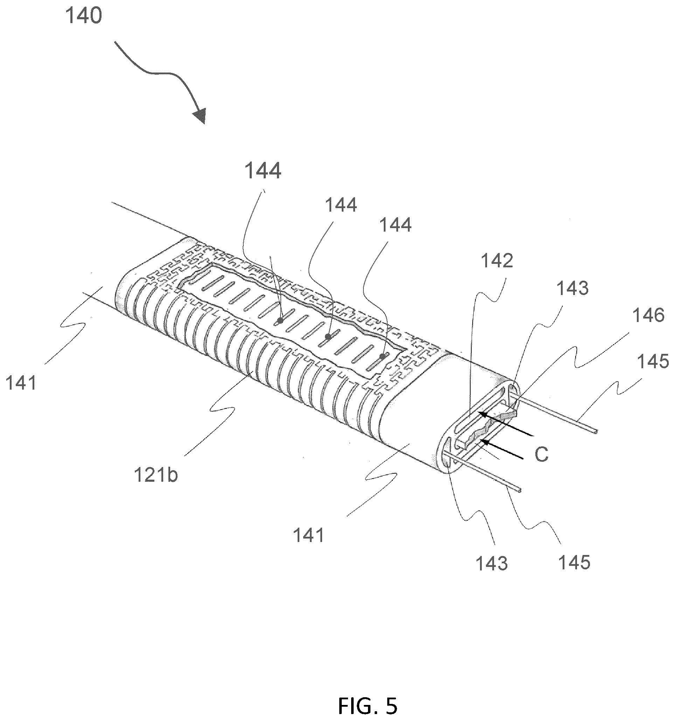

Fig. 5 partially show for example and without limitation that each of the plurality of flexibly railings 140 is provided with a plurality of holes 144 on the upper and lower portion of the sleeve 141, aligned with the integrated electrode 120, to allow irrigation through the electrode 120.

Advantageously the sleeve 141 has a multi-lumen profile with two or more lumens 142 to pass irrigation (C), two or more lumens 143 for conductor a wire and a central lumen 143 for structural support 146 of each of the plurality of flexibly railings 140, allowing that the plurality of flexible and irrigated electrodes 120 have an irrigation throughout the ablation procedure.

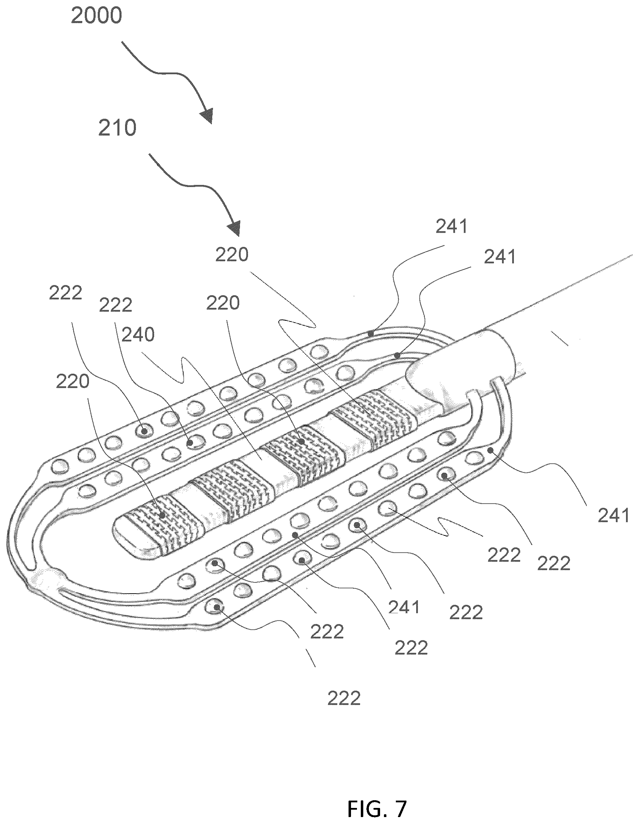

According to third embodiment (see Fig.6 and Fig.7) the catheter-based system 2000 comprises: at least a center flexibly railing 240 provided with the plurality of flexible and irrigated electrodes 220; and two or more flexible railings 241 provided of a plurality of button electrodes 222, allowing modular combinations of mapping and ablation contacts.

Advantageously the central flexibly rail supports the plurality of flexible and irrigated electrodes 220 which function as ablation-only electrodes and the two or more flexible railings 241 support a plurality of button electrodes 222 which function as mapping-only electrodes, enabling spatial functional segregation.

With non limiting reference to Figs. 6 and 7 each of two or more flexible railings 241 is disposed laterally and parallel to at least the central flexible railing 240.

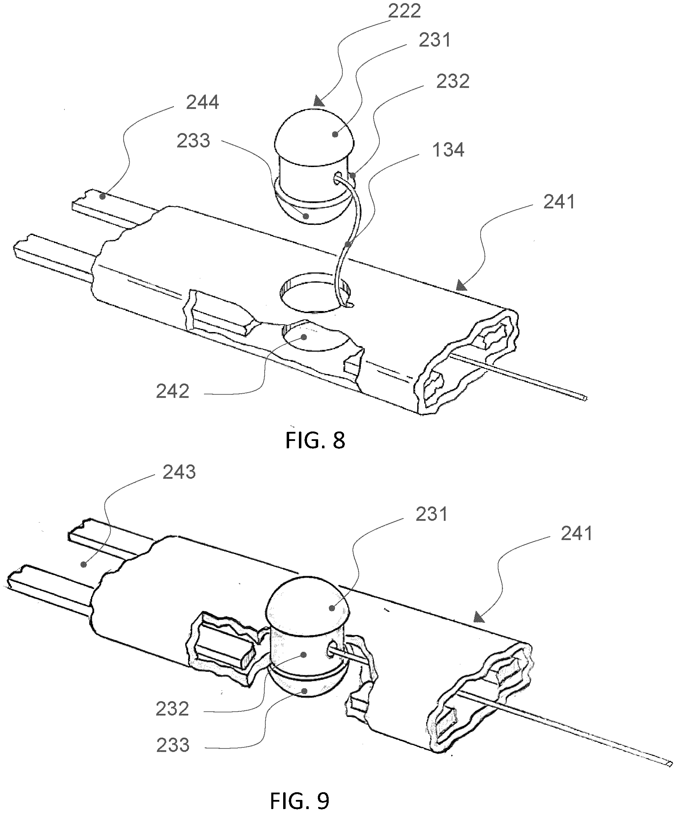

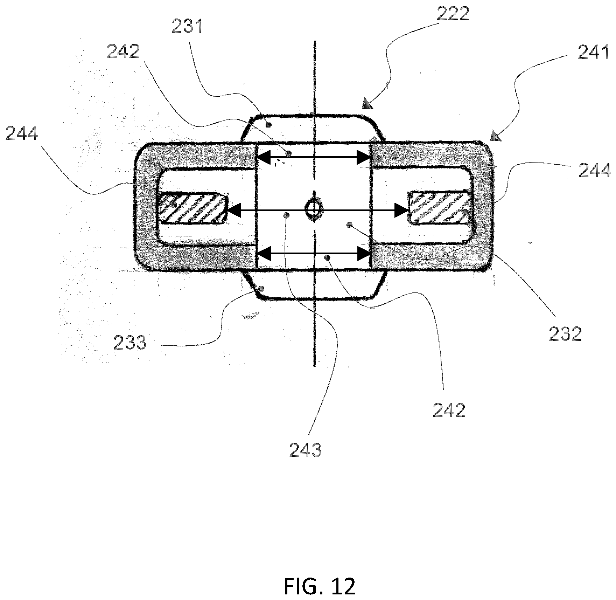

Advantageously, as shown in Figs. 8 and 9, each of two or more flexible railings 241 comprises at least a flexible hole 242 dimensionally smaller to the physical size of a slot 243 of an internal structure support 244, facilitating the insertion and fixing of each of the plurality of electrodes in at least the flexible hole 242 as shown in Fig. 12.

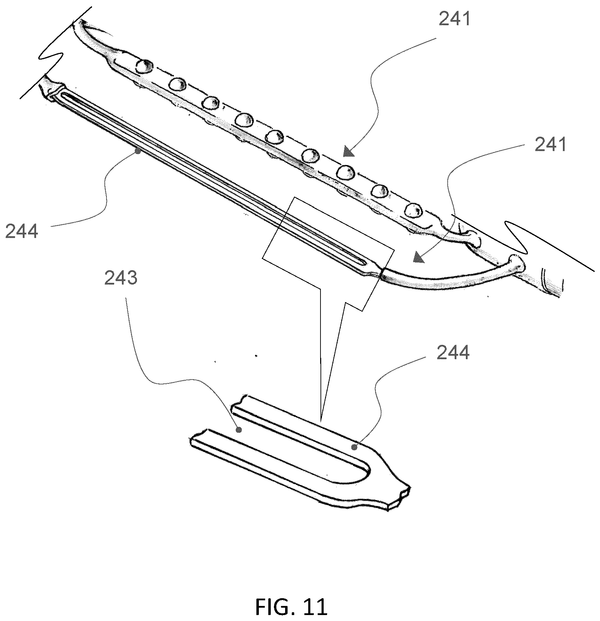

Advantageously shown in Fig. 11 two or more flexible railings 241 comprises at least the internal structure support 244 is provided with the slot 243 arranged longitudinally and centrally along each of the two or more flexible railings 241.

Each of the plurality of button electrodes 222 comprises an upper portion 231, a central portion 232, and a lower portion 233 wherein the upper portion 231 and the lower portion 233 have larger physical dimensions than the central portion 232 to locking central portion of each of the button electrodes 222 in at least the flexible hole 242 as shown in Fig. 12

Moreover, the upper portion 231 and the lower portion 233 protrude outward relative to the internal structure support 244 two or more flexible railings 241, when each of the plurality of button electrodes 222 is connected to two or more flexible railings 241.

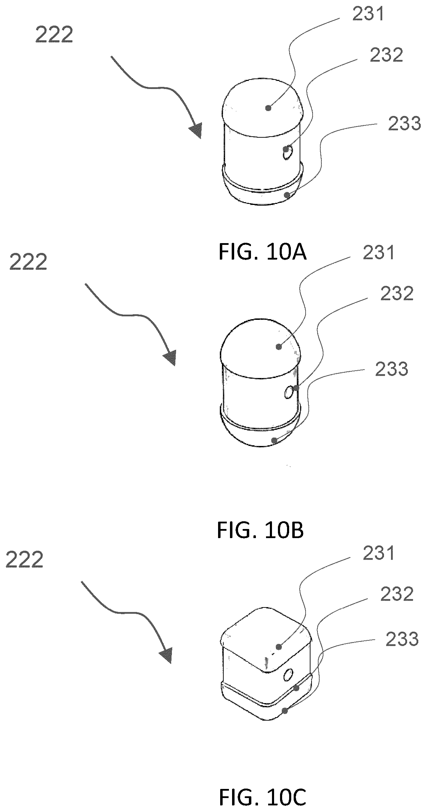

Figs. 10A, 10B, and 10C illustrates that the upper portion 231 and the lower portion 233 are a round portion, or a truncated dome portion, or a truncated square portion.

In addition, the plurality of button electrodes 222 has a circular or rectangular shape and is disposed along each of two or more flexible railings 241 in a linear configuration.

In addition, the catheter-based system 1000, 2000 is equipped with an impulse generator able of modulating the intensity and frequency of impulses emitted by the electrodes of the plurality of railings, by switching from emitting short pulses of high-voltage energy to pulses of low-voltage energy and the reverse.

Finally, the catheter-based system 1000, 2000 includes a proximal portion, the middle portion, and the distal portion with the therapeutic ablator device that are connected in sequence, wherein the therapeutic ablator device is connected to the middle portion through a manifold.

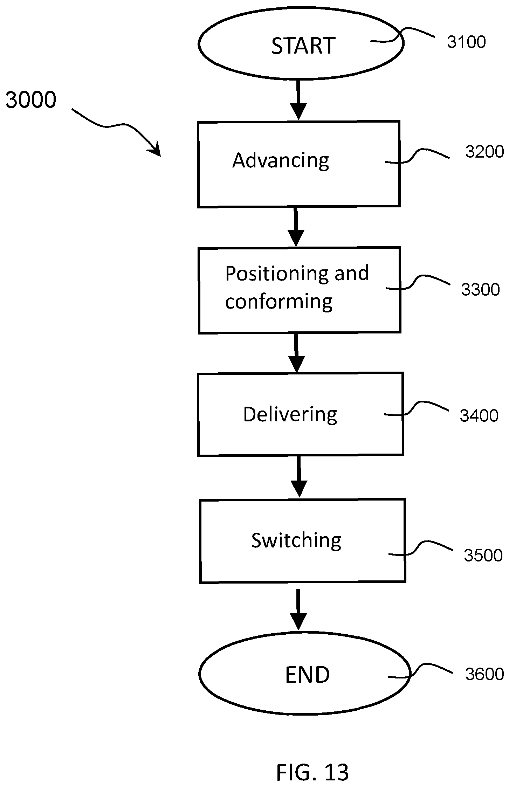

Fig. 13 illustrates a flow chart 3000 of an innovative method of use of a catheter-based system, for mapping and ablating a cardiac or epicardial target area of a patient to be treated, wherein the catheter-based system comprising a catheter including a mapping and ablator device. The flow chart has a start block 3100 and an end block 3600; the steps corresponding to block 3200 to block 3500 are typically repeated several times. Advantageously, the method 3000 comprising the steps of:
- advancing 3200 the catheter into a cardiac chamber or epicardial space
- positioning and conforming 3300 the mapping and ablator device to a cardiac or epicardial target area ;
- delivering 3400 energy through a plurality of flexible and irrigated electrodes and simultaneously acquiring electrograms ;
- switching 3500 between a radiofrequency (RF) generator and a pulsed field (PFA) generator based on data collected and processed by a control unit, such as data related to substrate response, clinical judgment, or tissue response.

The catheter-based system described herein is suitable for a variety of clinical applications including but not limited to:
- simultaneous high-density mapping and ablation during complex atrial and ventricular arrhythmia procedures.
- epicardial substrate mapping and ablation in patients with Brugada Syndrome, Long-QT syndrome, ARVC, early repolarization syndrome, or other cardiogenetic disorders.
- left and right atrial linear ablation (e.g., roof, anterior line, mitral isthmus) for treatment of atrial fibrillation and macro reentrant atrial tachycardias.
- ablation of the cavo tricuspid isthmus (CTI) for treatment of typical atrial flutter.
- mapping and modification of scar-related ventricular tachycardia substrates, both endocardially and epicardially.
- mapping and ablation of idiopathic or non-scar-related VT, including outflow tract and fascicular arrhythmias.
- real-time dynamic mapping and ablation in post-surgical or post-ablation atrial arrhythmias, including atypical atrial flutter and surgical scar-related tachycardias.
- mapping and therapeutic targeting of epicardial fat in HFpEF and atrial fibrillation, with irrigation-enhanced electrode-tissue interface and cooling.
- hybrid epicardial-endocardial procedures in complex ventricular or atrial substrates where transmurality and spatial orientation are key.
- experimental or translational research in EP, immunology, and cardio-oncology to study fibrosis, inflammation, or tumor-related myocardial alterations.
- lesion durability is enhanced through irrigation-cooled ablation and conforming contact geometry of the flexible railings.

## Claims

1. A catheter-based system (1000, 2000,) configured for simultaneous mapping and ablation of at least a target area of a cardiac tissue wherein the catheter based-system (1000, 2000,) comprises:
- a catheter (100, 200), including a mapping and ablator device (110, 210) disposed in a distal portion of the catheter,
- a radiofrequency (RF) generator (200);
- a pulsed field (PFA) generator (300);
- a control unit (400) configured to switch between the radiofrequency (RF) generator and the pulsed field ablation (PFA) generator,
wherein the mapping and ablator device (110, 210) comprises a plurality of flexible and irrigated electrodes (120, 220).

2. The catheter-based system (1000) of claim 1, wherein the mapping and ablator device (110) comprises a plurality of flexibly railings (140) arranged symmetrically and parallel to the axis(A) of the catheter (100) wherein the plurality of flexible and irrigated electrodes (120) disposed along the plurality of flexibly railings (140).

3. The catheter-based system (1000) of claim 2, wherein plurality of flexibly railings (140) is two flexibly railings (140a) arranged symmetrically and parallel to the axis(A)of the catheter (100) wherein the plurality of flexible and irrigated electrodes (120) disposed along two plurality of flexibly railings (140a).

4. The catheter-based system (1000) of claim 1, wherein each of the plurality of flexible and irrigated electrodes (120) has at least a cut on the outer surface of each electrode (120) to promote to promote flexibility and allow irrigation of each electrode (120).

5. The catheter-based system (1000) of claim 4, wherein the shape of at least the cut flows a repeating geometric pattern (121a) on the bottom portion of the outer surface of each electrode (120).

6. The catheter-based system (1000) of claim 4, wherein the shape of at least the cut flows a linear pattern (121b) on the lateral portion of the outer surface of each electrode (120).

7. The catheter-based system (1000) of claim 5, wherein the geometric pattern (121a) is helical or H shaped.

8. The catheter-based system (1000) of claim 4, wherein at least the cut of the plurality of flexible and irrigated electrodes (120) have a diameter d with 0,40≤d≤ 1mm.

9. The catheter-based system (1000) of claim 1, wherein the plurality of flexible and irrigated electrodes (120) has a stadium or semi-flat cross section.

10. The catheter-based system (1000) of claim 2, wherein the plurality of flexibly railings (140) has a stadium or semi-flat cross section, enhancing conformance to epicardial tissue.

11. The catheter-based system (1000) of claims 2 and 9, wherein each of the plurality of flexibly railings (140) provided with a plurality of holes (144) on the upper and lower portion of the sleeve (141), aligned with the integrated electrode (120), to allow irrigation through the electrode (120).

12. The catheter-based system (1000) of claim 11, wherein the sleeve (141) has a multi-lumen profile with two or more lumens (142) to pass irrigation (C), two or more lumens (143) for conductor a wire (145) and a central lumen (143) for structural support (146) of each of the plurality of flexibly railings (140).

13. The catheter-based system (2000) of claim 1, comprising:
- at least a center flexibly railing (240) provided with the plurality of flexible and irrigated electrodes (220); and
- two or more flexible railings (241) provided of a plurality of button electrodes (222), allowing modular combinations of mapping and ablation contacts.

14. The catheter-based system (2000) of claim 13, wherein each of two or more flexible railings (241) is disposed laterally and parallel to at least the central flexible railing (240).

15. The catheter-based system (2000) of claim 13, wherein each of two or more flexible railings (241) comprises at least a flexible hole (242) dimensionally comparable to the physical size of a slot (243) of a internal structure support (244), facilitating the insertion and fixing of each of the plurality of electrodes (222) in at least the flexible hole (242).

16. The catheter-based system (2000) of claim 13 and 15, wherein each of the plurality of button electrodes (222) comprises an upper portion (231), a central portion (232), and a lower portion (233) wherein the upper portion (231) and the lower portion(233) have larger physical dimensions than the central portion to locking central portion of each of the button electrodes (222) in the slot (243) of the internal structure support (244).

17. The catheter-based system (2000) of claim 16, wherein the upper portion (231) and the lower portion (233) protrude outward relative to the internal structure support (244) two o more flexible railings (241), when each of the plurality of button electrodes (222) is connected to two o more flexible railings (241)

18. The catheter-based system (2000) of claim 16, wherein the upper portion (231) and the lower portion (233) are a round portion, or a truncated dome portion, or a truncated square portion.

19. The catheter-based system (2000) of claim 13, wherein the plurality of button electrodes (222) has a circular or rectangular shape and is disposed along each of the plurality of railings (120) in a linear configuration.

20. A method (3000) of use of a catheter-based system according to anyone of claims 1 to 19 for mapping and ablating a cardiac or epicardial target area of a patient to be treated, wherein the catheter-based system comprising a catheter including a mapping and ablator device, wherein the method (3000) comprising the steps of:
- advancing (3200) the catheter into a cardiac chamber or epicardial space
- positioning and conforming (3300) the mapping and ablator device to a cardiac or epicardial target area ;
- delivering (3400) energy through a plurality of flexible and irrigated electrodes and simultaneously acquiring electrograms ;
- switching (3500) between a radiofrequency (RF) generator and a pulsed field (PFA) generator based on data collected and processed by a control unit.
